# EUROPEAN PATENT SPECIFICATION

(11) **EP 3 468 542 B1**
(45) Date of publication and mention of the grant of the patent: **26.05.2021**
(21) Application number: 17742852.1
(22) Date of filing: 09.06.2017
(51) Int. Cl.: A61K 31/05, A61K 9/107, A61K 9/14, A61K 9/16, A61K 31/202, A61K 31/593, A61K 36/324, A61K 36/9066

(54) **SELF EMULSIFYING AND GASTRO-RESISTANT FAT BASED MATRIX AND PREPARATION METHOD THEREOF**
SELBSTEMULGIERENDE UND MAGENSAFTRESISTENTE FETTBASIERTE MATRIX UND HERSTELLUNGSVERFAHREN DAFÜR
MATRICE À BASE DE GRAISSE AUTO-ÉMULSIFIANTE ET GASTRO-RÉSISTANTE ET SON PROCÉDÉ DE PRÉPARATION

(30) Priority: 09.06.2016 IT UA20164228
(43) Date of publication of application: 17.04.2019
(73) Proprietor: Labomar S.p.A., 31036 Istrana (TV) (IT)
(72) Inventor: BERTIN, Walter, 31036 Istrana TV (IT); FRATTER, Andrea, 31036 Istrana TV (IT)
(74) Representative: Perani & Partners S.p.A.
(86) International application number: PCT/IB2017/053416
(87) International publication number: WO 2017/212447

(56) References cited:
- WO-A1-00/09093
- WO-A1-2012/033478
- WO-A2-01/76561
- WO-A2-2011/161501
- WO-A2-2015/193380
- US-B2- 7 025 992
- B. V. Rajesh ET AL: "LIPID BASED SELF-EMULSIFYING DRUG DELIVERY SYSTEM (SEDDS) FOR POORLY WATER-SOLUBLE DRUGS: A REVIEW", Journal of Global Pharma Technology, 1 January 2009 (2009-01-01), pages 47-55, XP055025732, Retrieved from the Internet: URL:https://www.researchgate.net/publicati on/282312348_Lipid_based_self-emulsifying_ drug_delivery_system_SEDDS_for_poorly_wate r-soluble_drugs_A_review [retrieved on 2012-04-26]
- GUPTA SHWETA ET AL: "Formulation strategies to improve the bioavailability of poorly absorbed drugs with special emphasis on self-emulsifying systems.", ISRN PHARMACEUTICS 26 DEC 2013, vol. 2013, 848043, 26 December 2013 (2013-12-26), page 16PP, XP002767351, ISSN: 2090-6145, DOI: 10.1155/2013/848043

## Description

### Field of the invention

The present invention relates to an oral pharmaceutical formulation for the delivery of lipophilic, gastro-damaging and/or unpleasantly tasting active principles and related preparation method.

### State of the art

Omega-3 fatty acids (Omega-3) are essential fatty acids, structurally constituted by a long-chain carboxylic acid characterized by several unsaturations. These components are called essential because they are crucial for the health of the organism, performing relevant functions for the cell membrane, as precursors of cardiovascular chemical mediators, in blood fluidisation and as an important energy reserve. Despite the importance of the above components, human organism is not able to synthesize them autonomously and must take them from the outside, through a balanced diet or by taking supplements/integrators.

Several nutraceutical products based on Omega-3 are presently available on the market, but they are mostly characterized by a single formulation, namely the soft capsule.

For their chemical structure, polyunsaturated fatty acids are liquid at room temperature and hardly deliverable in a solid pharmaceutical form like hard tablets or capsules. The use of soft capsules is the most obvious choice in the delivery of these active principles, but its several technological drawbacks can negatively affect the formulation. In fact, polyunsaturated fatty acids are known to be susceptible to oxidation, which is a process responsible for the rancidity of the active principle as well as for the reduction of the biological activity of the components. Moreover, if compared with a coated tablet or a hard capsule, soft capsules do not completely hinder the passage of oxygen through the formulation, thus causing an alteration of the taste and of the smell of the product over time.

Furthermore, soft capsules have a limited masking power, therefore hardly covering the unpleasant taste intrinsically related to some of the active principles (fish oils). Beside Omega-3, another product class that has been a great success in the nutraceutical industry is the one of phytotherapeutic remedies, active principles of vegetable origin that are used for the prevention or the treatment of various disorders of human organism, alternatively or in combination with "traditional" pharmacological therapies.

Many of these molecules, precisely because of their vegetable origin, have a considerable structural complexity, hardly reproducible in the laboratory and equally difficult to deliver with the presently available formulation tools. One of the most common problems is the poor solubility in water of the aforesaid active principles, since they are characterized by a high molecular weight and/or by a certain level of lipophilicity when they are not already present in nature in the form of oils (Serenoa Repens, vitamin A, E, D, K) or lipophilic terpenes such as boswellic acids and others.

In all these cases, the high lipophilicity complicates the delivery in the classical forms of tablets and capsules as well as in aqueous liquids and, moreover, makes difficult and incomplete the intestinal assimilation occurring after the dissolution or the emulsification of the active principle in the aqueous enteral secretions.

Besides having a difficult delivery due to their poor solubility in water, substances of fatty/lipophilic nature even hinder the production of solid pharmaceutical forms that should contain them, especially when they must be delivered in large amounts.

Said fatty/lipophilic substances tend to grease the surface of the production machinery, such as e.g. the punches of the compressing machines, making it difficult to check the uniformity and dosage of the final pharmaceutical form, which must adhere to demanding quality standards, and furthermore do not let the tablets reach the minimum hardness that guarantees their integrity within the blister packs. Selecting a formulation therefore becomes highly relevant, both from a productive-technological point of view and from a pharmacological point of view, since the lack of dissolution of the active principle may be the cause of the ineffectiveness of the product.

The high tolerability of natural active principles does not exclude the presence of undesired effects, especially in the gastro-intestinal tract, where nausea, abdominal pain and gastric intolerance can be particularly frequent.

Therefore, the delivery strategy has a key role not only in ensuring the curing power of the integrator/supplement, but also in limiting those side effects that can reduce the patient compliance, such as in particular gastro-irritation, sense of nausea and sickness.

Moreover, self-emulsifying fat matrix formulations for oral formulations of liposoluble active principles are known from the state of the art, such as those described in WO12/033478, in which Marijuana extracts are dispersed.

Besides containing a monoglyceride of a fatty acid and ascorbyl palmitate, these fat matrices contain another component that seems to be the only responsible for the self-emulsifying properties, namely a triglyceride of a fatty acid such as Cremophor RH40.

Moreover, the presence of a solvent whose purpose is dissolving the active principle is essential in the preparatory phase.

In any case, the final formulations inserted in hard capsules are never in the form of a powder, but look like a liquid or semi-solid material.

Also the formulations disclosed in US7025992 are very similar to those described in the previous prior art documents.

In fact, also this patent discloses a self-emulsifying fat matrix comprising ascorbyl palmitate and a monoglyceride of a fatty acid, namely the mono glyceryl stearate in which a lipophilic active principle is dispersed.

However, the solid oral formulation is prepared with a known technique encompassing the indispensable presence of an organic solvent (ethanol), which disperses and/or dissolves the active principle, mono glyceryl stearate and ascorbyl palmitate. The thus obtained solution/dispersion is sprayed on the solid powder, or lactose, and the obtained mixture is subsequently compressed.

This type of tablets is in any case suitable for the mucosal, but not systemic, oral administration.

US7025992 also contemplates formulations in the form of soft capsules that, once chewed, turn into non-aqueous liquid solutions. Moreover, this type of formulation allows the absorption of the active principle in any gastrointestinal tract.

Also WO2015/193380 describes oral formulations in the form of tablets or capsules containing self-emulsifying mixtures comprising ascorbyl palmitate and monoglycerides.

The tablets are prepared by adsorbing on already formed tablets, consisting only of conventional excipients, a self-emulsifying liquid matrix containing the active principle, while the capsules contain only the oily liquid phase that is inserted in the hard capsules.

### Summary of the invention

The Applicant has now surprisingly found that a high amount of lipophilic molecules, even in the form of oils, having gastro-irritant properties or an unpleasant taste or both, can be conveyed in a formulation comprising a self-emulsifying and gastro resistant fat matrix, which can obviate the problems of bioavailability, gastric damage and poor patient compliance known in the state of the art.

Moreover, due to the particular self-emulsifying fat matrix, these formulations are easily deliverable with suitable excipients, thus forming powders that are compressible or are suitable to be inserted in hard capsules or in aqueous suspensions.

The object of the present invention is therefore an oral formulation selected between:
(i) a solid form, as a compressible or a flowable powder that can be inserted in hard capsules, or
(ii) a liquid form, as an aqueous suspension
comprising one or more lipophilic active principle, being gastro-irritating or unpleasantly tasting or both, dispersed in a self-emulsifying and gastro resistant fat matrix (A), which in turn comprises:
- mono and diglycerides of fatty acids C12-C20,
- an ascorbic acid ester with a fatty acid C12-C20,
wherein said one or more active principles are liquid and/or solid at room temperature and are dispersed in said self-emulsifying and gastro resistant fat matrix (A) with a process consisting of the following steps:a) dry mixing the mixture of mono and diglycerides of fatty acids C12-C20, the ascorbic acid ester with a fatty acid C12-C20 and the one or more active principles if they are solid
b)hot melting the mixture obtained in step a),
c) adding the active principle to the molten mass in case said one or more active principles are liquid at room temperature.

The formulation object of the present invention has good organoleptic characteristics of gastro-resistance and enteric emulsification.

A further object of the present invention are methods for the preparation of the formulation in the compressible solid form or as a powder to be inserted in hard capsules and in the liquid form of a suspension.

### Description of the figures

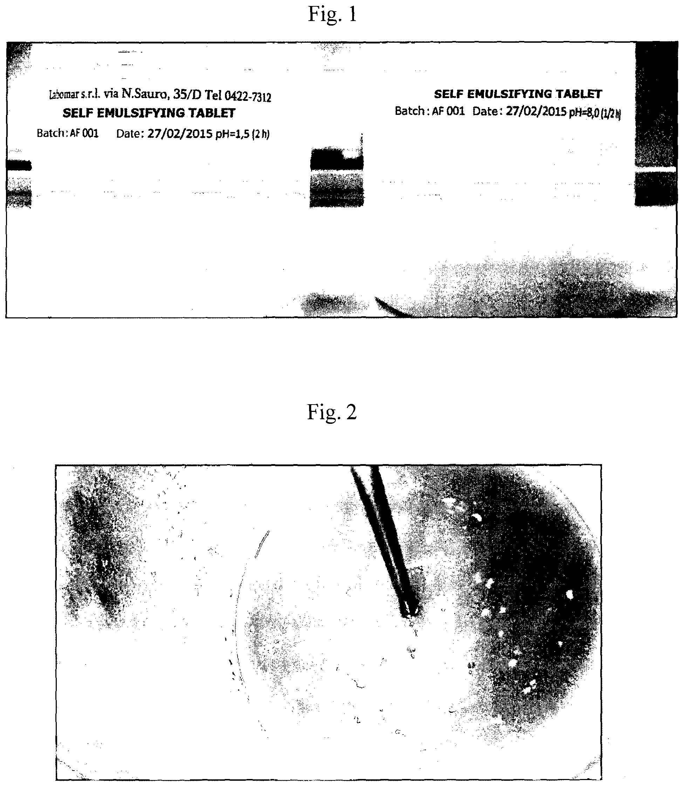

Figure 1 shows what obtained by dissolving a self-emulsifying tablet, prepared by direct compression of the powder object of the invention containing the tracer copper chlorophyll, an extract of Boswellia Serrata titrated at 75% in boswellic acids and at 10% in alpha-keto-boswellic acid (AKBA).

The solution on the left shows the result of the immersion of self-emulsifying tablets in a liquid simulating the acidic environment of the stomach (pH=1.5) for hydrochloric acid. The solution on the right shows instead what obtained from the hydro-dispersion of the same self-emulsifying tablets, directly taken from the beaker containing the solution of hydrochloric acid at pH=1.5, in a liquid simulating the enteric environment (pH=8.0) for sodium bicarbonate and in the presence of 5% w/v sodium glycocholate. A complete breakdown and hydro-dispersion of the solution occurs in 30 minutes.

Figure 2 shows the liquid suspension obtained by adding dropwise the self-emulsifying, gastro resistant hot fat matrix (t=75°C) containing 99% resveratrol from Polygonum Cuspidatum root and black pepper fruit titrated at 95% in pipeline, to the cold gelled aqueous liquid (T=10°C). The image clearly shows the sudden coarse solidification of the dispersed lipophilic phase in contact with the cold aqueous matrix.

Figure 3 shows the liquid suspension obtained by adding dropwise the self-emulsifying, gastro resistant hot fat matrix, to the cold, gelled liquid after homogenizing by means of a turboemulsifier-homogenizer the dispersed phase.

Figure 4 shows the liquid formulation exemplified in Example 4.

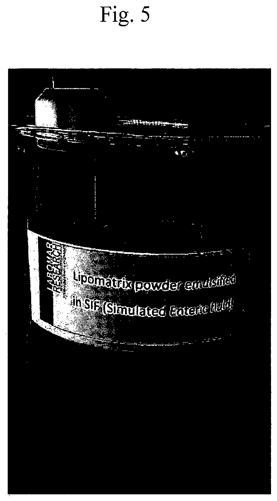

Figure 5 shows, instead, the formation of the emulsion of the liquid suspension when it encounters the enteric liquid SIF heated at 37°C.

### Detailed description of the present invention

For the purposes of the present invention, the term lipophilic active principle means a molecule with specific biological properties, characterized by a poor solubility in aqueous medium and by a low enteric bioavailability in vivo.

Active gastro-irritating principle means a molecule whose biological properties are responsible for a specific therapeutic effect as well as for an unwanted irritation of the gastric mucosa, because of a systemic or local effect, such as e.g. some inorganic ferrous salts and in particular ferrous sulphate.

Unpleasantly tasting active principle means a molecule with specific biological properties, tasting unpleasantly or in any case tasting so that it is difficult to take it as it is.

For the purposes of the present invention, self-emulsifying gastro resistant fat matrix means a mixture mainly consisting of lipophilic molecules comprising components with surfactant properties, spontaneously emulsifying in an enteric aqueous medium (pH>7) at a temperature higher than 36°C and selectively disaggregating at enteric level, passing unchanged through the stomach.

The self-emulsifying gastro resistant fat matrix (A) preferably comprises from 0.1 to 30% by weight of mono and diglycerides of fatty acids C12-C20, from 0.1 to 30% by weight of ascorbic acid ester with a fatty acid C12-C20 on the total weight of the formulation.

For the purposes of the present invention, the ascorbic acid ester with a fatty acid C12-C20 is preferably ascorbyl palmitate.

In an alternative embodiment, the self-emulsifying gastro resistant fat matrix (A) contained in the formulation object of the present invention further contains a hydrophilic emulsifier with a high HLB (HLB: Hydrophilic-Lipophilic Balance) >9 and preferably in an amount comprised between 0.1 and 10% by weight based on the total weight of the formulation.

Said hydrophilic emulsifier is preferably selected among a polyoxyethylene sorbitan ester with a fatty acid C12-C20, a sucrose ester with a fatty acid C12-C20 (sucroester) or mixtures of the two.

The fatty acids C12-C20 contained in the formulation of the present invention, when in the form of ascorbic acid esters, sucrose esters or mono and diglycerides, are preferably selected among saturated fatty acids C12-C20.

The fatty acids C12-C20 contained in the formulation of the present invention, when in the form of polyoxyethylene sorbitan esters, may be selected among saturated or unsaturated fatty acids C12-C20.

The preferred polyoxyethylene sorbitan esters with a fatty acid C12-C20 according to the purposes of the present invention are polysorbate 20 and polysorbate 80.

The self-emulsifying gastro resistant fat matrix (A) contained in the formulation object of the present invention has peculiar self-emulsifying characteristics, which allow it to convey poorly soluble molecules through the formation of hydrophilic micelles in an enteric aqueous environment at pH > 7.

The peculiar self-emulsifying characteristics are related to the presence of the ascorbic acid ester with a fatty acid C12-C20, whose amphoteric characteristics allow an easy solubilisation of the molecule in the self-emulsifying gastro resistant fat matrix (A) while applying a surfactant power responsible for the emulsification of insoluble active principles once in contact with the enteral alkaline aqueous secretions by sodium bicarbonate.

In an alkaline aqueous environment (pH = 8.0 - 8.5) by sodium bicarbonate secreted in pancreatic juice, the ascorbic acid ester with a fatty acid C12-C20 becomes hydrosoluble after ionisation of the acid enediol function and produces foam, with the consequent transformation into an ionic surfactant.

Together with mono and diglycerides C12-C20, and possibly together with a hydrophilic emulsifier when present, the ascorbic acid ester with a fatty acid C12-C20 forms hydrophilic mixed micelles having fat-emulsifying properties in an aqueous environment, especially when the pH is higher than 7 (duodenal environment), thus promoting the hydro-dispersion of the active principles encapsulated in the micelles and their contextual better absorption in the intestinal epithelium.

The hydrophilic emulsifier, when present, synergistically works with the ascorbic acid ester with a fatty acid C12-C20, the glycerides of fatty acids and the bile salts present at enteric level, thus contributing to the micellization of the active principles and increasing their permeability in the intestinal wall.

The same mechanism that promotes self-emulsification therefore has the further function of allowing the selective release of the active principle at intestinal level, where the formulation is dispersed in micellar form and releases the conveyed active principle.

The formulation object of the present invention shows characteristics of gastro-resistance and improved bioavailability of lipophilic active principles through an effective emulsification triggered by the same excipients in an alkaline environment. The formulation object of the invention is refractory to the contact with the hydrochloric acid of gastric juices, thereby reaching unchanged the intestinal cavern. The formulation object of the present invention is preferably in solid form or in the form of a liquid suspension.

For the purposes of the present invention, formulation in solid form means a compressible powder or a powder for filling hard capsules.

Compressible powder means a solid formulation, such as a powder, which can generate compact materials having a suitable hardness, preferably not less than 9 kg, even more preferably comprised between 14 and 18 kg.

For the purposes of the present invention, powder for filling capsules means a powder with such flowability characteristics and chemical-physical parameters to be correctly processed in the machinery for filling capsules.

When in solid form, the formulation object of the present invention comprises the self-emulsifying gastro resistant fat matrix (A) and a solid phase (B) comprising polyols and/or sugars.

The solid phase (B) includes polyols and/or sugars in an amount preferably ranging from 10 to 65% by weight based on the total weight of the formulation.

The polyols and/or sugars contained in said solid phase (B) are preferably selected among at least one of the following: mannitol, xylitol, sorbitol, inositol, isomalt, sucrose, fructose and dextrose.

Advantageously, the formulation object of the present invention enables the preparation of solid pharmaceutical forms, such as tablets, provided with a good hardness, preferably comprised between 12 and 15 kg (Durometer).

This technological requirement is hardly obtainable from formulations containing a high fat dosage (up to 25-30% w/w) and in particular from compositions containing liquid or semisolid active principles such as polyunsaturated fatty acids and plant sterols.

In a particularly preferred embodiment, said solid phase (B) further contains an ascorbic acid ester with a fatty acid C12-C20, preferably in an amount ranging from 0.1% to 30% by weight based on the total weight of the formulation.

The addition of an ascorbic acid ester with a fatty acid C12-C20 to the solid phase (B) advantageously improves the uniformity of the hardness of the tablets over time. The formulation object of the present invention, when in solid form, is preferably used for the preparation of pharmaceutical forms for the oral use of a conventional type, such as for example tablets and capsules, wherein the formulation of interest is associated with suitable excipients and diluents.

According to a particularly preferred embodiment, the solid pharmaceutical forms obtained with the solid formulation described herein are prepared with the aid of excipients commonly used for oral solid forms, such as amorphous silica, calcium phosphate, magnesium stearate.

When in the form of a drinkable liquid aqueous suspension (ii), the formulation object of the present invention comprises the self-emulsifying gastro resistant fat matrix (A) suspended in a liquid phase (B') comprising water and a gelling hydrophilic polymer.

Preferably, said liquid phase (B') comprises from 70% to 99% by weight of water and from 0.05% to 2% by weight of a gelling hydrophilic polymer, and optionally glycerol.

When said glycerol is present, it is preferably contained in an amount ranging from 1% to 10% by weight based on the total weight of the formulation.

Before being dispersed in the aqueous phase (B'), especially when the active principle has a particularly unpleasant taste, the liquid suspension is sometimes a suspension of a powder containing the fat matrix (A) as well as the same polyols and/or sugars used for the preparation of the solid formulation (i).

In this case, the content of polyol and/or sugar in the aqueous dispersion ranges from 5% to 20% of the total weight of the liquid formulation.

Preferably, the gelling hydrophilic polymer is, for example, selected among xanthan gum, sodium hyaluronate, guar gum, cellulose derivatives etc.

Advantageously, the self-emulsifying and gastro resistant technology of the formulation object of the present invention allows conveying unpleasantly tasting active principles by effectively covering their taste, thus increasing the patient compliance.

The form of liquid suspension of the formulation object of the present invention guarantees an excellent organoleptic coverage of unpleasantly tasting active principles, while maintaining the gastro resistant and enteric self-emulsifying characteristics.

Due to these self-emulsifying properties, the active principles deliverable according to the formulation object of the present invention are lipophilic molecules whose poor solubility in water and, consequently, difficult absorption, have the final effect of a low bioavailability and minimum therapeutic activity.

Another category of substances which are particularly suited to the delivery by means of the above technology are gastro-irritating substances including, in particular, ferrous salts, with a particular reference to ferrous sulphate heptahydrate and liposoluble vegetable fractions with gastro-irritating action (essential oils, terpenes and others).

In parallel, the gastro resistant properties of the formulation in question lead to considerable technological advantages, since the possibility of protecting the stomach from the damaging action of gastro-irritating active principles improves the risk/benefit ratio of the pharmaceutical product, as well as the patient compliance.

In the preferred embodiment, the formulation object of the present patent application contains active principles selected from the group consisting of: plant sterols; Boswellia Serrata D.E. (dry extract); Omega-3 fatty acids; ferrous sulphate and other ferrous salts; Turmeric D.E. (turmeric dry extract); resveratrol; liposoluble vitamins (vitamin D3, vitamin K and its vitamers, vitamin A and E); palmitoyl ethanolamide; gastro-irritating and scarcely bio-available nutraceutical or pharmaceutical substances.

For the purposes of the present invention, vitamers mean all those chemical compounds that, although structurally different, have a biological activity attributable to the same parent vitamin.

For the purposes of the present invention, nutraceutical refers to a "food-drug", i.e. a food that associates nutritional components selected for their characteristics, such as high digestibility and hypoallergenicity, to the curing properties of natural active principles.

In a preferred embodiment, the formulation according to the present invention comprises Serenoa Repens titrated in total sterols, preferably in the form of dried extracts or oils.

In the preferred embodiment, the formulation object of the present invention comprises as active principle Boswellia Serrata D.E. titrated in boswellic acids.

The formulation object of the present invention is preferably used in the preparation of food supplements, foods for special medical purposes, functional foods, medical devices, medicinal products.

For the purposes of the present invention, food supplements indicate specific products favouring the taking of certain active principles when the body needs them or in case of a wrong diet.

For the purposes of the present invention, foods for special medical purposes mean food intended for patients who have nutritional needs that cannot be satisfied by normal food because of a disease, a disorder or a particular medical condition.

### (https://www.efsa.europa.eu/it_{/}press/news/151126)

For the purposes of the present invention, a food is functional if it satisfactorily demonstrates to have positive effects beyond normal nutritional effects on one or more specific functions of the organism that make it relevant for improving the health and the well-being and/or for reducing any risk of disease.

### (http://www.eufic.org/article/it/expid/basics-alimenti-funzionali/)

Medical device means any instrument, apparatus and substance, used alone or in combination, for diagnostic and/or therapeutic purposes.

Medicinal products mean industrially produced pharmaceutical forms containing at least a pharmaceutical active principle for the treatment and/or the prevention of a disease in animals and, even more preferably, in humans.

The formulation object of the present invention is obtained with a process that is characterized by the fact that the active principle is dissolved in the melted fat coating matrix without requiring the addition of solvents as it occurs with the formulations disclosed in the aforesaid prior art.

In this way, the active principle is evenly dispersed in a fat matrix with surfactant and gastro resistant characteristics conferred by the glycerides, by the ascorbic acid ester with a fatty acid C12-C20 and by a possible further hydrophilic surfactant agent.

The molten mass is subsequently cooled under distinct operating conditions depending on whether you want to obtain the solid formulation (i) or the liquid formulation (ii).

A further object of the present are the different methods that allow obtaining both the solid (i) and the liquid (ii) formulations.

The preparation method of the formulation according to the present invention in solid form (i) comprises the following steps:
a) dry mixing mono and diglycerides of fatty acids C12-C20, an ascorbic acid ester with a fatty acid C12-C20 and the active principle if this latter is solid;
b) hot melting the fat mixture referred to in step a) at a temperature comprised between 70°C and 85°C until obtaining a molten fat mass;
c) adding an active principle to the molten fat mass from step b), if this latter is liquid at room temperature;
d) providing a solid phase comprising polyols and/or sugars;
e) cooling the solid phase of step c) at a temperature between 5°C and 10°C;
f) joining the molten fat mass from step c) to the solid phase referred to in step e) until complete solidification of the molten mass and simultaneous formation of the powder.

The hot molten fat mixture (70°C < T < 85°C), when joined to the cold solid phase (5°C < T < 10°C) comprising polyols and/or sugars, is instantaneously solidified through intimate adsorption on said cold solid phase to produce an even, gastro resistant, self-emulsifying powder in an enteric and compressible alkaline environment.

At the same time, after solidification, it is formed an ordered crystal lattice in which the active principle is evenly dispersed.

At this point, the formulation object of the invention can be used for obtaining the pharmaceutical form of interest through compression or encapsulation.

The compression or encapsulation methods usable for manufacturing solid pharmaceutical forms comprising the formulation of the present invention are the classical ones, known to those skilled in the art and disclosed in the inherent technical literature *(*Remington: The Science and Practice of Pharmacy, 21st edition, Lippincott Williams & Wilkins).

In an alternative embodiment, the preparation method of the formulation in solid form contgemplates that an aliquot of the ascorbic acid ester with a fatty acid C12-C20, preferably ascorbyl palmitate, is mixed with polyols and sugars in step d) to form the solid phase of the formulation of interest before that the molten fat mass from step c) is poured on said solid phase previously cooled in step e).

A further object of the present patent application is a preparation method of the formulation according to the present invention in the form of a drinkable liquid suspension, comprising the following steps:
a. dry mixing mono and diglycerides of fatty acids, an ascorbic acid ester with a fatty acid C12-C20 and the active principle, if this latter is solid at room temperature;
b. hot melting the fat mixture referred to in step a. at a temperature comprised between 70°C and 85°C until obtaining a molten fat mass;
c. adding an active principle to the molten fat mass from step b. if this latter is liquid at room temperature;
d. providing an aqueous liquid phase comprising a gelling hydrophilic polymer and optionally supplemented with glycerol from 1% to 10% by weight;
e. cooling the aqueous liquid phase referred to in step d. at a temperature comprised between 5°C and 10°C;
f. joining dropwise under stirring the molten fat mass from step c. to the aqueous liquid phase referred to in step e. until complete solidification by crystallization of the molten fat mass and simultaneous formation of the coarse suspension.

Once having joined the whole molten fat mass to the cold aqueous phase and having verified the successful solidification of the molten fat mass, the size of the lipid droplets is reduced by means of a turboemulsifier-homogenizer which makes the suspension even and free of coarse particulate (Fig. 2).

In case the formulation contains polyols and/or sugars, the process for the preparation of the liquid formulation (ii) comprises the following steps:
a. dry mixing mono and diglycerides of fatty acids of an ascorbic acid ester with a fatty acid C12-C20 and the active principle if this latter is solid at room temperature;
b. hot melting the fat mixture referred to in step a) at a temperature comprised between 70°C and 85°C until obtaining a molten fat mass;
c. adding an active principle to the molten fat mass from step b if this latter is liquid at room temperature;
d. joining the molten mass to the polyol and/or sugar cooled at a temperature comprised between 5°C and 10°C and obtaining a powder,
e. dispersing the powder in an aqueous liquid phase comprising a gelling hydrophilic polymer and optionally supplemented with glycerol from 1% to 10% by weight.

In a preferred embodiment, the aqueous liquid phase of the liquid formulations (ii) according to the present invention is supplemented with a hydrophilic flavouring agent.

In the formulation in the suspension liquid phase, also the fat phase can be usefully supplemented with a lipophilic aroma for better fixing the final taste of the liquid.

In an even more preferred mode, the preparation method of the formulation in the solid form (i) or in the liquid form (ii) of aqueous suspension provides that the monoglycerides and diglycerides of fatty acids C12-C20 and the ascorbic acid ester with a fatty acid C12-C20 are melted together in step (a) with a polyoxyethylene sorbitan ester with a fatty acid C 12-C20 or a sucrose ester with a fatty acid C 12-C20 or a mixture of the two.

Unexpectedly, in this case the melting occurs more quickly and at a lower temperature.

For purely illustrative and not limitative purposes, examples relating to the preparation of oral formulations of the present invention, both in the solid form (i) and in the liquid form (ii) of suspension are reported hereinafter.

### Example 1

Table 1 lists the concentrations by weight/weight of the components forming a powder consisting of the formulation object of the present invention in solid form.

**Table 1**

| **Phase** | **Component** | **Concentration weight/total weight composition** |
|---|---|---|
| Fat matrix | Mono and diglycerides of fatty acids | 18.00 % |
| | Ascorbyl palmitate | 0.10 % |
| | Boswellia Resin 75% boswellic acids | 22.25 % |
| | Vitamin D3 | 0.05 % |
| Solid phase | Ascorbyl palmitate | 23.00 % |
| | Mannitol | 17.00 % |
| | Magnesium stearate | 4.00 % |
| | Amorphous silica | 3.00 % |
| | Dibasic calcium phosphate dihydrate | 12.60 % |

The pharmaceutical powder is obtained by the process described hereinafter.
A. Dry mixing ascorbyl palmitate and mannitol.
B. Cooling in a refrigerator the mixture obtained from A. up to a temperature of 10°C.
C. Dry mixing mono and diglycerides of fatty acids, ascorbyl palmitate and Boswellia.
D. Hot melting the mixture obtained from C. at a temperature between 70°C and 80°C.
E. Stabilizing the temperature of the phase obtained in D. at 80°C and adding the vitamin D3.
F. Immediately after, gradually adsorbing under stirring the fat liquid phase obtained in E. in the cold mixture obtained in B.
G. Cooling the powder obtained in F. and sieving.
H. Adding the magnesium stearate, the amorphous silica and the dibasic calcium phosphate dihydrate to the powder obtained in G.

### Example 2

Table 2 shows an example of the preparation of a pharmaceutical powder consisting of the formulation object of the present invention in solid form.

**Table 2**

| **Phase** | **Component** | **Concentration weight/total weight composition** |
|---|---|---|
| Fat matrix | Mono and diglycerides of fatty acids | 16.00 % |
| | Ascorbyl palmitate | 8.00 % |
| | Polysorbate 80 | 3,00 % |
| | Vitamin E | 5.52 % |
| | Vitamin A | 0.23 % |
| | Vitamin K1 | 0.01 % |
| | Vitamin D3 | 0,10 % |
| Solid phase | Mannitol | 57,14 % |
| | Magnesium Stearate | 4,00 % |
| | Amorphous silica | 6,00 % |

The pharmaceutical powder relating to Example 2 is obtained by the process described hereinafter.
I. Cooling the mannitol in a refrigerator up to a temperature of 10°C.
II. Mixing mono and diglycerides of fatty acids, ascorbyl palmitate and polysorbate 80.
III. Hot melting the mixture obtained in II. at a temperature between 70°C and 80°C.
IV. Mixing vitamin E, vitamin A, vitamin K1 and vitamin D3.
V. Stabilizing the temperature of the mixture obtained in III. at 80°C and adding the mixture obtained in IV.
VI. Immediately after, gradually adsorbing under stirring the fatty liquid phase obtained in V. in cold mannitol.
VII. Cooling the powder obtained in VI. and sieving.
VIII. Adding the magnesium stearate and the amorphous silica to the powder obtained in VII.

### Example 3:

Table 3 shows an example of embodiment of the formulation in the form of a gastro resistant and self-emulsifying drinkable liquid obiect of the present invention.

**Table 3**

| **Phase** | **Component** | **Concentration weight/total weight composition** |
|---|---|---|
| Fat matrix | Mono and diglycerides of fatty acids | 1.50 % |
| | Ascorbyl palmitate | 1.20 % |
| | Caprylic and capric triglyceride | 4.00 % |
| | Sucrester | 060 % |
| | 99% Resveratrol from Poligonum Cuspidatum | 0.30 % |
| | Black pepper fruit 95 % piperine | 0.01 % |
| | Cream favour | 0.20 % |
| Liquid Phase | Purified water | 86.42 % |
| | Xanthan gum | 0.27 % |
| | Glycerine | 4.50 % |
| | Potassium sorbate | 0.19 % |
| | Grapefruit seed 50% Bioflavonoids | 0.60 % |
| | Sucralose | 0.024 % |
| | Acesulfame-K | 0.028 % |
| | Cream Caramel flavour | 0.15 % |

The liquid suspension is obtained by the process described hereinafter.
1. Dissolving under stirring the potassium sorbate in purified water.
2. Adding grapefruit dry extract to the solution obtained in 1.
3. Moistening xanthan gum with glycerine.
4. Pouring under stirring the mixture obtained in 3 in the solution obtained in 2.
5. Cooling the aqueous phase referred to in step 4 to a temperature ranging from 5°C to 10°C.
6. Dry mixing mono and diglycerides of fatty acids, ascorbyl palmitate, caprylic/capric triglyceride, sucrester and resveratrol.
7. Hot melting the mixture obtained in 6 at a temperature between 70°C and 80°C.
8. Immediately before joining the phases, adding black pepper to the mixture obtained in 7.
9. Immediately before joining the phases, adding the cream flavour to the aqueous phase from step 5.
10. Joining dropwise under stirring the molten fat mass obtained in 8 to the aqueous liquid phase referred to in step 9.
11. Reducing the size of the lipid droplets by means of a turboemulsifier-homogenizer.
12. Adding sucralose, acesulfame-K and cream caramel flavour to the suspension obtained in 11.

### Example 4

It is prepared a liquid formulation containing the following components

**Table 4**

| **Phase** | **Component** | |
|---|---|---|
| Fat matrix | Mono and diglycerides of fatty acids | 0.300 g |
| | Ascorbyl palmitate | 0.050 g |
| | Boswellia Resin 75% boswellic acids | 0.250 g |
| | Vitamin D3 | 25 mcg (1000 IU) |
| Solid phase | Ascorbyl palmitate | 0.075 g |
| | Mannitol | 0.600 g |
| Liquid Phase | Purified water | q.s. to 100 g |
| | Xanthan gum | 0.250 g |
| | Glycerine | 5.000 g |
| | Potassium sorbate | 0.200 g |

The formulation given above in Table 4 was prepared according to the following operating modes:
A. Dry mixing ascorbyl palmitate and mannitol.
B. Cooling in a refrigerator the mixture obtained in A. up to a temperature of 10°C.
C. Dry mixing mono and diglycerides of fatty acids, ascorbyl palmitate and Boswellia.
D. Hot melting the mixture obtained in C. at a temperature between 70°C and 80°C.
E. Immediately after, gradually adsorbing under stirring the fat liquid phase obtained in E. in the cold mixture obtained in B, thus obtaining a powder.
F. Dissolving potassium sorbate in water.
G. Moistening xanthan gum with glycerine.
H. Pouring under stirring the mixture from G. in the solution obtained in F, thus obtaining a gelled aqueous phase.
I. Dispersing the powder from step E in the aqueous liquid phase from step H.

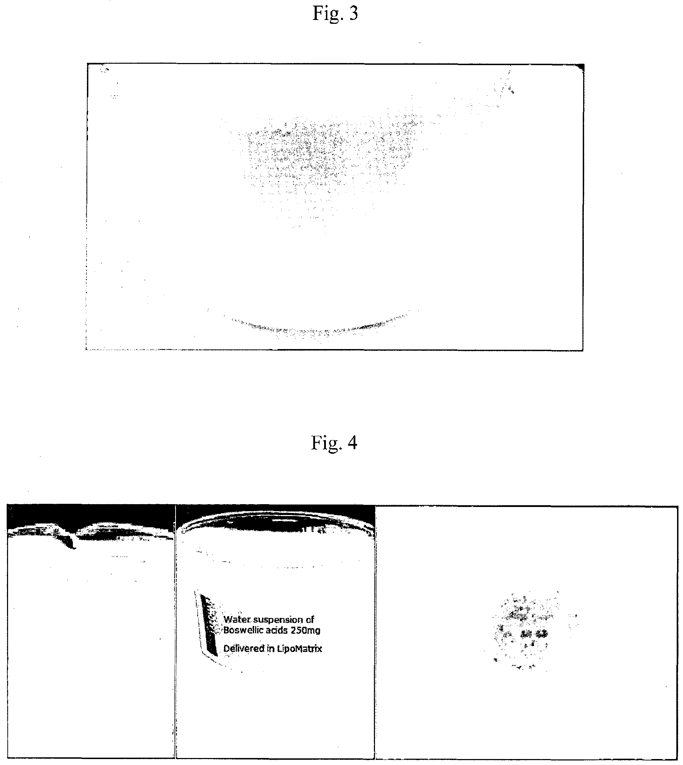

It is evident the formation of a solid suspension stabilized by the gelling agent in the aqueous phase (xanthan gum or other polysaccharides) at 25°C as shown in Figure 4.

### Preliminary Test

For illustrative purposes only, it was carried out a preliminary dissolution test of standard tablets obtained with the formulation object of the present patent application, in solid form (i), and in liquid form (ii), to demonstrate the self-emulsifying and gastro protective properties claimed in the above patent.

The tablets prepared as described in the Example 1 and the liquid formulation of the Example 4 are prepared by adding to the coating fat matrix the lipophilic tracer copper chlorophyll, capable of diffusing and colouring the surrounding aqueous system only in case of efficient emulsification.

The carrier powder copper chlorophyll is prepared according to the preparation method object of the present invention, according to the aforesaid steps I-VIII where the tracer, insoluble in water, is added as if it were the active principle.

The thus obtained powder was used to prepare the chlorophyll-based tablets, which were immersed in a solution at pH 1.5 for hydrochloric acid.

The solution was kept under magnetic stirring at a thermostatically controlled temperature of 37°C for 2 hours, in order to simulate the gastric environment.

At the end of the two hours, part of the liquid was poured in an alkaline solution at pH 8.0 for sodium bicarbonate, together with some tablets. The remaining tablets were left in the acid solution, by means of comparison.

In order to reproduce the environment of the duodenal cavern, the alkaline liquid is supplemented with 5% of glycocholic acid, a bile salt released at enteric level and present in the form of sodium salt for the emulsification of fats.

The solution is kept under moderate magnetic stirring for 30 minutes, at a temperature of 37°C.

The solutions are visually inspected.

As shown by the picture in Figure 1, the acid solution is clear, showing no sign of the tracer in the liquid.

The tablets have also kept their shape, only showing a minimal surface dusting, probably produced by the collision of the magnetic stir bar with the tablet, but no breakdown or dissolution phenomena.

The solution on the right, the one simulating the alkaline environment of the first enteric tract, is coloured green, indicating the breakdown of the tablet and the completed emulsification of the lipophilic tracer.

This simple preliminary test therefore shows not only the inertia of the tablet to the action of the hydrochloric acid, but also the actual emulsification of the alkaline pH preparation, with a dispersion of the active principle (the lipophilic tracer copper chlorophyll) in the aqueous medium. Indirectly, it also proves the effective compressibility of the powder and the possibility of obtaining a tablet despite the high fat content of the formulation.

Analogously, also the stable liquid suspension of Example 4 turns into a coloured emulsion when contacted by the simulated enteric liquid (SIF) at 37°C.

The suspended powders in the liquid formulation of the Example 4 retain inside them the active principle, but only in a liquid containing bile salts and having a pH higher than 7.5, which is the pH at which ascorbyl palmitate starts ionising, the suspension becomes emulsion and makes available the active principle for the absorption only at the intestinal level. The emulsification of said powder also occurs in response to the enteral lipases that cleave the glycerides, thus increasing the surfactant power of the powder.

## Claims

1. Oral formulation selected between:
(i) a solid form, as a compressible or a flowable powder that can be inserted in hard capsules, or
(ii) a liquid aqueous suspension
comprising:
• one or more lipophilic and gastro-irritating active principles,
• one or more lipophilic and unpleasantly tasting active principles, or
• one or more lipophilic gastro-irritating and unpleasantly tasting active principles, dispersed in a self-emulsifying and gastro resistant fat matrix (A), which in turn comprises:
- mono and diglycerides of fatty acids C12-C20,
- an ascorbic acid ester with a fatty acid C 12-C20,
wherein said one or more active principles are liquid or solid at room temperature and are dispersed in said self-emulsifying and gastro resistant fat matrix (A) with a process consisting of the following steps:
a) dry mixing the mixture of mono and diglycerides of fatty acids C12-C20, the ascorbic acid ester with a fatty acid C12-C20 and the one or more active principles if they are solid,
b) hot melting the mixture obtained in step a),
c) adding the active principle to the molten mass in case said one or more active principles are liquid at room temperature.

2. Oral formulation according to claim 1, wherein when said formulation is of type (i), it comprises a solid phase (B) comprising polyols and/or sugars, when it is of type (ii) it comprises a liquid phase (B') comprising water and a gelling hydrophilic polymer.

3. Oral formulation according to claim 1 or 2, wherein the self-emulsifying gastro resistant fat matrix (A) comprises from 0.1% to 30% by weight of mono and diglycerides of fatty acids C12-C20, from 0.1% to 30% by weight of ascorbic acid ester with a fatty acid C12-C20 on the total weight of the formulation.

4. Formulation according to any one of claims 1 to 3, wherein the self-emulsifying gastro resistant fat matrix (A) contains a hydrophilic emulsifier selected among those with HLB>9.

5. Formulation according to claim 4, wherein said hydrophilic emulsifier is selected among a polyoxyethylene sorbitan ester with a fatty acid C12-C20, a sucrose ester with a fatty acid C12-C20 or mixtures of the two.

6. Formulation according to any one of claims 4 or 5, wherein said hydrophilic emulsifier is present in an amount comprised between 0.1% and 10% by weight based on the total weight of the formulation.

7. Oral solid formulation of type (i) according to any one of claims 2-5, wherein the solid phase (B) includes polyols and/or sugars selected among mannitol, xylitol, sorbitol, isomalt, inositol, sucrose, fructose and dextrose.

8. Oral solid formulation of type (i) according to any one of claims 2-7, wherein said polyols and/or sugars are present in an amount ranging from 10% to 65% by weight based on the total weight of the solid formulation (i).

9. Oral solid formulation of type (i) according to any one of claims 2 to 7, wherein also said solid phase (B) contains an ascorbic acid ester with a fatty acid C 12-C20.

10. Oral liquid formulation of type (ii) according to any one of claims 2 to 5, wherein the gelling hydrophilic polymer is selected among: xanthan gum, sodium hyaluronate, guar gum, cellulose derivatives.

11. Oral liquid formulation of type (ii) according to any one of claims 2 to 5 and 10, wherein said liquid phase (B') comprises glycerol.

12. Oral liquid formulation of type (ii) according to claim 10, wherein said liquid phase comprises from 70% to 99% by weight of water, from 0.05% to 2% by weight of a gelling hydrophilic polymer and from 1% to 10% of glycerol.

13. Oral liquid formulation (ii) according to claims 1, 10-12, wherein when the active principle has a particularly unpleasant taste, before being dispersed in the aqueous phase (B'), the liquid suspension is sometimes a suspension of a powder containing the fat matrix (A) as well as the same polyols and/or sugars used for the preparation of the solid formulation (i) of claim 2.

14. Oral formulation according to any one of claims 1 to 13, wherein said ascorbic acid ester with a fatty acid C12-C20 is ascorbyl palmitate.

15. Formulation according to any one of claims 1-14, wherein the poorly hydrosoluble and/or gastro-irritating and/or unpleasantly tasting active principle is preferably selected from the group consisting of: plant sterols; Boswellia Serrata D.E..; Omega-3 fatty acids; Turmeric D.E..; resveratrol; liposoluble vitamins (vitamin A, E, D3, K and its vitamers); palmitoyl ethanolamide; ferrous sulphate and other ferrous salts and their associations.

16. Method for the preparation of the oral solid formulation of type (i) according to any one of claims 1 to 9, 14 and 15, comprising the following steps:
a) dry mixing mono and diglycerides of fatty acids C12-C20 and an ascorbic acid ester with a fatty acid C12-C20 and the active principle if this latter is solid at room temperature;
b) hot melting the fat mixture referred to in step a) at a temperature comprised between 70°C and 85°C until obtaining a molten fat mass;
c) adding one or more active principles to the molten fat mass from step b), if this one or more active principles are liquid at room temperature;
d) providing a solid phase comprising polyols and/or sugars;
e) cooling the solid phase of step d) at a temperature between 5°C and 10°C;
f) joining the molten fat mass from step c) to the solid phase referred to in step e) under stirring until complete solidification by crystallization of the liquid mass and simultaneous formation of the homogeneous powder.

17. Method for the preparation of the oral liquid formulation of type (ii) according to any one of claims 1-5, 10-12, 14 and 15, comprising the following steps:
a) dry mixing mono and diglycerides of fatty acids, an ascorbic acid ester with a fatty acid C12-C20 and one or more active principles, if they are solid at room temperature;
b) hot melting the fat mixture referred to in step a) at a temperature comprised between 70°C and 85°C until obtaining a molten fat mass;
c) adding one or more active principles to the molten fat mass from step b) if said one or more active principles are liquid at room temperature;
d) providing an aqueous liquid phase comprising a gelling hydrophilic polymer and optionally glycerol;
e) cooling the aqueous liquid phase from step d) at a temperature comprised between 5°C and 10°C;
f) joining dropwise under stirring the molten fat mass from step c) to the aqueous liquid phase from step e) until complete solidification of the molten fat mass and simultaneous formation of the suspension.

18. Method for the preparation of the liquid formulation (ii) according to claim 13, comprising the following steps:
a) dry mixing mono and diglycerides of fatty acids, an ascorbic acid ester with a fatty acid C12-C20 and the active principle if this latter is solid at room temperature;
b) hot melting the fat mixture referred to in step a) at a temperature comprised between 70°C and 85°C until obtaining a molten fat mass;
c) adding an active principle to the molten fat mass from step b) if this latter is liquid at room temperature;
d) joining the molten mass on the polyol and/or sugar cooled at a temperature comprised between 5°C and 10°C,
e) dispersing the powder in an aqueous liquid phase comprising a gelling hydrophilic polymer and optionally supplemented with glycerol.

19. Formulation according to any one of claims 1 to 15 for use in the preparation of food supplements, foods for special medical purposes, functional foods, medical devices, medicinal products.

## Patentansprüche

1. Orale Formulierung ausgewählt aus:
(i) einer festen Form als komprimierbares oder fließfähiges Pulver, das in Hartkapseln eingesetzt werden kann, oder
(ii) einer flüssigen wässrige Suspension, umfassend:
• einen oder mehrere lipophile und gastro-irritirende Wirkstoffe,
• einen oder mehrere lipophile und unangenehm schmeckende Wirkstoffe oder
• einen oder mehrere lipophile gastro-irritierende und unangenehm schmeckende Wirkstoffe, dispergiert in einer selbstemulgierenden und gastro-resistenten Fettmatrix (A), die wiederum umfasst:
- Mono- und Diglyceride der Fettsäuren C12-C20,
- ein Ascorbinsäureester mit einer Fettsäure C12-C20,
wobei der eine oder die mehreren Wirkstoffe bei Raumtemperatur flüssig oder fest sind und in der selbstemulgierenden und gastro-resistenten Fettmatrix (A) mit einem Verfahren dispergiert sind, das aus den folgenden Schritten besteht:
a) Trockenmischen der Mischung aus Mono- und Diglyceriden der Fettsäuren C12-C20, des Ascorbinsäureesters mit einer Fettsäure C12-C20 und des einen oder der mehreren Wirkstoffs, wenn sie fest sind,
b) Heißschmelzen der in Schritt a) erhaltenen Mischung,
c) Zugeben des Wirkstoffs zu der geschmolzenen Masse, falls der eine oder die mehreren Wirkstoffe bei Raumtemperatur flüssig sind.

2. Orale Formulierung nach Anspruch 1, wobei, wenn die Formulierung vom Typ (i) ist, sie eine feste Phase (B) umfasst, die Polyole und/oder Zucker umfasst, wenn sie
Typ ist (ii) eine flüssige Phase (B') umfasst, die Wasser und ein gelierendes hydrophiles Polymer umfasst.

3. Orale Formulierung nach Anspruch 1 oder 2, wobei die selbstemulgierende gastro-resistente Fettmatrix (A) 0,1 bis 30 Gew.- % Mono- und Diglyceride von Fettsäuren C12-C20, 0,1 bis 30 Gew.- % Ascorbinsäureester mit einer Fettsäure C12-C20, auf das Gesamtgewicht der Formulierung, umfasst.

4. Formulierung nach einem der Ansprüche 1 bis 3, wobei die selbstemulgierende gastro-resistente Fettmatrix (A) einen hydrophilen Emulgator enthält, ausgewählt aus denjenigen mit HLB>9.

5. Formulierung nach Anspruch 4, wobei der hydrophile Emulgator ausgewählt ist aus einem Polyoxyethylensorbitanester mit einer Fettsäure C12-C20, einem Saccharoseester mit einer Fettsäure C12-C20 oder Mischungen aus beiden.

6. Formulierung nach einem der Ansprüche 4 oder 5, wobei der hydrophile Emulgator in einer Menge zwischen 0,1 Gew.- % und 10 Gew.- %, bezogen auf das Gesamtgewicht der Formulierung, vorhanden ist.

7. Orale feste Formulierung des Typs (i) nach einem der Ansprüche 2 bis 5, wobei die feste Phase (B) Polyole und/oder Zucker, ausgewählt aus Mannit, Xylit, Sorbit, Isomalt, Inosit, Saccharose, Fructose und Dextrose, einschließt.

8. Orale feste Formulierung vom Typ (i) nach einem der Ansprüche 2 bis 7, wobei die Polyole und/oder Zucker in einer Menge im Bereich von 10 bis 65 Gew.-%, gemessen am Gesamtgewicht der festen Formulierung (i), vorliegt.

9. Orale feste Formulierung vom Typ (i) nach einem der Ansprüche 2 bis 7, wobei auch die feste Phase (B) einen Ascorbinsäureester mit einer Fettsäure C12-C20 enthält.

10. Orale flüssige Formulierung vom Typ (ii) nach einem der Ansprüche 2 bis 5, wobei das gelierende hydrophile Polymer ausgewählt ist aus: Xanthangummi, Natriumhyaluronat, Guargummi, Cellulosederivaten.

11. Orale flüssige Formulierung vom Typ (ii) nach einem der Ansprüche 2 bis 5 und 10, wobei die flüssige Phase (B') Glycerin umfasst.

12. Orale flüssige Formulierung vom Typ (ii) nach Anspruch 10, wobei die flüssige Phase 70 bis 99 Gew.- % Wasser, 0,05 bis 2 Gew.- % eines gelierenden hydrophilen Polymers und 1 bis 10 Gew.- % Glycerin umfasst.

13. Orale flüssige Formulierung (ii) nach Anspruch 1, 10-12, wobei, wenn der Wirkstoff einen besonders unangenehmen Geschmack hat, bevor er in der wässrigen Phase (B') dispergiert wird, die flüssige Suspension manchmal eine Suspension eines Pulvers ist, das die Fettmatrix (A) sowie dieselben Polyole und/oder Zucker enthält, die bei der Herstellung der festen Formulierung (i) nach Anspruch 2 verwendet wurden.

14. Orale Formulierung nach einem der Ansprüche 1 bis 13, wobei der Ascorbinsäureester mit einer Fettsäure C12-C20 Ascorbylpalmitat ist.

15. Formulierung nach einem der Ansprüche 1-14, wobei der schlecht wasserlösliche und/oder gastro-irritierende und/oder unangenehm schmeckende Wirkstoff bevorzugt ausgewählt ist aus einer Gruppe bestehend aus: Pflanzensterolen; Boswellia Serrata D.E.; Omega-3-Fettsäuren; Kurkuma D.E.; Resveratrol; fettlöslichen Vitaminen (Vitamin A, E, D3, K und seinen Vitaminen); Palmitoylethanolamid; Eisen (II) sulfat und anderen Eisen (II) salzen und deren Verbindungen.

16. Verfahren zur Herstellung der oralen festen Formulierung des Typs (i) nach einem der Ansprüche 1 bis 9, 14 und 15, umfassend die folgenden Schritte:
a) Trockenmischen von Mono- und Diglyceriden der Fettsäuren C12-C20 und eines Ascorbinsäureesters mit einer Fettsäure C12-C20 und dem Wirkstoff, wenn letzterer bei Raumtemperatur fest ist;
b) Heißschmelzen der in Schritt a) genannten Fettmischung bei einer Temperatur zwischen 70 °C und 85 °C, bis eine geschmolzene Fettmasse erhalten wird;
c) Zugeben eines oder mehrerer Wirkstoffe zu der geschmolzenen Fettmasse aus Schritt b), sofern dieser eine oder diese mehreren Wirkstoffe bei Raumtemperatur flüssig sind;
d) Bereitstellen einer festen Phase, die Polyole und/oder Zucker umfasst;
e) Abkühlen der festen Phase von Schritt d) bei einer Temperatur zwischen 5 °C und 10 °C;
f) Zusammenfügen der geschmolzenen Fettmasse aus Schritt c) mit der in Schritt e) erwähnten festen Phase unter Rühren bis zum vollständigen Verfestigen durch Kristallisation der flüssigen Masse und gleichzeitiger Bildung des homogenen Pulvers.

17. Verfahren zur Herstellung der oralen flüssigen Formulierung des Typs (ii) nach einem der Ansprüche 1-5, 10-12, 14 und 15, das die folgenden Schritte umfasst:
a) Trockenmischen von Mono- und Diglyceriden von Fettsäuren, einem Ascorbinsäureester mit einer Fettsäure C12-C20 und einem oder mehreren Wirkstoffen, wenn sie bei Raumtemperatur fest sind;
b) Heißschmelzen der in Schritt a) genannten Fettmischung bei einer Temperatur zwischen 70 °C und 85 °C, bis eine geschmolzene Fettmasse erhalten wird;
c) Zugeben von einem oder mehrerer Wirkstoffe zu der geschmolzenen Fettmasse aus Schritt b), wenn der eine oder die mehreren Wirkstoffe bei Raumtemperatur flüssig sind;
d) Bereitstellen einer wässrigen flüssigen Phase, die ein gelierendes hydrophiles Polymer und wahlweise Glycerin umfasst;
e) Abkühlen der wässrigen flüssigen Phase aus Schritt d) bei einer Temperatur zwischen 5 °C und 10 °C;
f) Tropfenweises hinzufügen der geschmolzenen Fettmasse aus Schritt c) unter Rühren in die wässrige flüssige Phase aus Schritt e) bis zur vollständigen Verfestigung der geschmolzenen Fettmasse und gleichzeitigen Ausbildung der Suspension.

18. Verfahren zur Herstellung der flüssigen Formulierung (ii) nach Anspruch 13, umfassend die folgenden Schritte:
a) Trockenmischen von Mono- und Diglyceriden von Fettsäuren, eines Ascorbinsäureesters mit einer Fettsäure C12-C20 und des Wirkstoffs, wenn letzteres bei Raumtemperatur fest ist;
b) Heißschmelzen der in Schritt a) genannten Fettmischung bei einer Temperatur zwischen 70 °C und 85 °C, bis eine geschmolzene Fettmasse erhalten wird;
c) Zugabe eines Wirkstoffs zu der geschmolzenen Fettmasse aus Schritt b), wenn diese bei Raumtemperatur flüssig ist;
d) Verbinden der geschmolzenen Masse mit dem Polyol und/oder Zucker, der bei einer Temperatur zwischen 5 °C und 10 °C gekühlt wird,
e) Dispergieren des Pulvers in einer wässrigen flüssigen Phase, umfassend ein gelierendes hydrophiles Polymer und wahlweise ergänzt mit Glycerin.

19. Formulierung nach einem der Ansprüche 1 bis 15 zur Verwendung bei der Herstellung von Nahrungsergänzungsmitteln, Lebensmitteln für spezielle medizinische Zwecke, funktionellen Lebensmitteln, medizinischen Vorrichtungen, medizinischen Produkten.

## Revendications

1. Formulation orale choisie entre:
(i) une forme solide, une forme de poudre compressible ou fluide pouvant être insérée dans des capsules dures, ou
(ii) une suspension aqueuse liquide comprenant :
• un ou plusieurs principes actifs lipophiles et gastro-irritants,
• un ou plusieurs principes actifs lipophiles au goût désagréable, ou
• un ou plusieurs principes actifs lipophiles gastro-irritants et au goût désagréable, dispersés dans une matrice grasse auto-émulsifiante et gastro-résistante (A), qui comprend à son tour :
- des mono- et diglycérides d'acides gras C12-C20,
- un ester d'acide ascorbique avec un acide gras C12-C20,
où ledit ou lesdits principes actifs sont liquides ou solides à température ambiante et sont dispersés dans ladite matrice grasse auto-émulsifiante et gastro-résistante (A) avec un procédé consistant en les étapes suivantes :
a) mélanger à sec le mélange de mono- et diglycérides d'acides gras C12-C20, l'ester d'acide ascorbique avec un acide gras C12-C20 et le ou les principes actifs s'ils sont solides,
b) faire fondre à chaud le mélange obtenu à l'étape a),
c) ajouter le principe actif à la masse fondue dans le cas où ledit ou lesdits principes actifs sont liquides à température ambiante.

2. Formulation orale selon la revendication 1, où, lorsque ladite formulation est de type (i), elle comprend une phase solide (B) comprenant des polyols et/ou des sucres, lorsqu'elle est de
type (ii) elle comprend une phase liquide (B') comprenant de l'eau et un polymère hydrophile gélifiant.

3. Formulation orale selon la revendication 1 ou 2, où la matrice grasse gastro-résistante auto-émulsifiante (A) comprend de 0,1% à 30% en poids de mono- et diglycérides d'acides gras C12-C20, de 0,1% à 30% en poids d'ester d'acide ascorbique avec un acide gras C 12-C20 sur le poids total de la formulation.

4. Formulation selon l'une quelconque des revendications 1 à 3, où la matrice grasse gastro-résistante auto-émulsifiante (A) contient un émulsifiant hydrophile choisi parmi ceux avec HLB>9.

5. Formulation selon la revendication 4, où ledit émulsifiant hydrophile est choisi parmi un ester de polyoxyéthylène sorbitane avec un acide gras C12-C20, un ester de saccharose avec un acide gras C12-C20 ou des mélanges des deux.

6. Formulation selon l'une quelconque des revendications 4 ou 5, où ledit émulsifiant hydrophile est présent en une quantité comprise entre 0,1% et 10% en poids sur la base du poids total de la formulation.

7. Formulation solide orale de type (i) selon l'une quelconque des revendications 2 à 5, où la phase solide (B) comprend des polyols et/ou des sucres choisis parmi le mannitol, le xylitol, le sorbitol, l'isomalt, l'inositol, le saccharose, le fructose et le dextrose.

8. Formulation solide orale de type (i) selon l'une quelconque des revendications 2 à 7, où lesdits polyols et/ou sucres sont présents en une quantité allant de 10% à 65% en poids sur la base du poids total de la formulation solide (i).

9. Formulation solide orale de type (i) selon l'une quelconque des revendications 2 à 7, où également ladite phase solide (B) contient un ester d'acide ascorbique avec un acide gras C12-C20.

10. Formulation liquide orale de type (ii) selon l'une quelconque des revendications 2 à 5, où le polymère hydrophile gélifiant est choisi parmi : la gomme xanthane, l'hyaluronate de sodium, la gomme guar, les dérivés de cellulose.

11. Formulation liquide orale de type (ii) selon l'une quelconque des revendications 2 à 5 et 10, où ladite phase liquide (B') comprend du glycérol.

12. Formulation liquide orale de type (ii) selon la revendication 10, où ladite phase liquide comprend de 70% à 99% en poids d'eau, de 0,05% à 2% en poids d'un polymère hydrophile gélifiant et de 1% à 10% de glycérol.

13. Formulation liquide orale (ii) selon les revendications 1, 10 à 12, où, lorsque le principe actif a un goût particulièrement désagréable, avant d'être dispersée dans la phase aqueuse (B'), la suspension liquide est parfois une suspension d'une poudre contenant la matrice grasse (A) ainsi que les mêmes polyols et/ou sucres utilisés pour la préparation de la formulation solide (i) selon la revendication 2.

14. Formulation orale selon l'une quelconque des revendications 1 à 13, où ledit ester d'acide ascorbique avec un acide gras C12-C20 est le palmitate d'ascorbyle.

15. Formulation selon l'une quelconque des revendications 1 à 14, où le principe actif faiblement hydrosoluble et/ou gastro-irritant et/ou au goût désagréable est de préférence choisi dans le groupe constitué par : les stérols végétaux ; Boswellia Serrata D.E. ; les acides gras oméga-3 ; le curcuma D.E. ; le resvératrol ; les vitamines liposolubles (vitamine A, E, D3, K et ses vitamères) ; le palmitoyléthanolamide ; le sulfate ferreux et d'autres sels ferreux et leurs associations.

16. Procédé de préparation de la formulation solide orale de type (i) selon l'une quelconque des revendications 1 à 9, 14 et 15, comprenant les étapes suivantes :
a) mélanger à sec des mono- et diglycérides d'acides gras C12-C20 et d'un ester d'acide ascorbique avec un acide gras C12-C20 et le principe actif si ce dernier est solide à température ambiante ;
b) faire fondre à chaud le mélange de matières grasses visé à l'étape a) à une température comprise entre 70°C et 85°C jusqu'à obtention d'une masse grasse fondue ;
c) ajouter un ou plusieurs principes actifs à la masse grasse fondue de l'étape b), si ce ou ces principes actifs sont liquides à température ambiante ;
d) fournir une phase solide comprenant des polyols et/ou des sucres ;
e) refroidir la phase solide de l'étape d) à une température comprise entre 5°C et 10°C ;
f) joindre la masse grasse fondue de l'étape c) à la phase solide visée à l'étape e) sous agitation jusqu'à solidification complète par cristallisation de la masse liquide et formation simultanée de la poudre homogène.

17. Procédé de préparation de la formulation liquide orale du type (ii) selon l'une quelconque des revendications 1 à 5, 10 à 12, 14 et 15, comprenant les étapes suivantes :
a) mélanger à sec des mono- et diglycérides d'acides gras, un ester d'acide ascorbique avec un acide gras C12-C20 et un ou plusieurs principes actifs, s'ils sont solides à température ambiante ;
b) faire fondre à chaud le mélange de matières grasses visé à l'étape a) à une température comprise entre 70°C et 85°C jusqu'à obtention d'une masse grasse fondue ;
c) ajouter un ou plusieurs principes actifs à la masse grasse fondue de l'étape b) si lesdits un ou plusieurs principes actifs sont liquides à température ambiante ;
d) fournir une phase liquide aqueuse comprenant un polymère hydrophile gélifiant et éventuellement du glycérol ;
e) refroidir la phase liquide aqueuse de l'étape d) à une température comprise entre 5°C et 10°C ;
f) joindre goutte à goutte sous agitation la masse grasse fondue de l'étape c) à la phase liquide aqueuse de l'étape e) jusqu'à solidification complète de la masse grasse fondue et formation simultanée de la suspension.

18. Procédé de préparation de la formulation liquide (ii) selon la revendication 13, comprenant les étapes suivantes :
a) mélanger à sec des mono- et diglycérides d'acides gras, un ester d'acide ascorbique avec un acide gras C12-C20 et le principe actif si ce dernier est solide à température ambiante ;
b) faire fondre à chaud le mélange de matières grasses visé à l'étape a) à une température comprise entre 70°C et 85°C jusqu'à obtention d'une masse grasse fondue ;
c) ajouter un principe actif à la masse grasse fondue de l'étape b) si cette dernière est liquide à température ambiante ;
d) joindre la masse fondue sur le polyol et/ou le sucre refroidi à une température comprise entre 5°C et 10°C,
e) disperser la poudre dans une phase liquide aqueuse comprenant un polymère hydrophile gélifiant et éventuellement complété par du glycérol.

19. Formulation selon l'une quelconque des revendications 1 à 15, destinée à être utilisée dans la préparation de compléments alimentaires, d'aliments à des fins médicales spéciales, d'aliments fonctionnels, de dispositifs médicaux, de médicaments.
